# FASCICULE DE BREVET EUROPEEN

(11) **EP 3 600 010 B1**
(45) Date de publication et mention de la délivrance du brevet: **18.06.2025**
(21) Numéro de dépôt: 18722096.7
(22) Date de dépôt: 28.03.2018
(51) Int. Cl.: A61B 5/00, A61B 5/024

(54) **SYSTEME DE DETERMINATION D'UN ENSEMBLE D'AU MOINS UN DESCRIPTEUR CARDIO-RESPIRATOIRE D'UN INDIVIDU PENDANT SON SOMMEIL**
SYSTEM ZUR BESTIMMUNG EINES SATZES AUS MINDESTENS EINEM KARDIORESPIRATORISCHEN DESKRIPTOR EINER PERSON WÄHREND DES SCHLAFENS
SYSTEM FOR DETERMINING A SET OF AT LEAST ONE CARDIO-RESPIRATORY DESCRIPTOR OF AN INDIVIDUAL DURING SLEEP

(30) Priorité: 30.03.2017 FR 1752688
(43) Date de publication de la demande: 05.02.2020
(73) Titulaire: Université Grenoble Alpes, 38400 Saint-Martin-d'Hères (FR)
(72) Inventeur: COLAS, Damien, 69300 Caluire-Et-Cuire (FR); BRICOUT, Aurélien, 69002 LYON (FR); GERARD, Grégoire, 69160 Tassin La Demi Lune (FR); GUMERY, Pierre-Yves, 38000 Grenoble (FR)
(74) Mandataire: Bouvier, Thibault
(86) Numéro de dépôt international: PCT/FR2018/050753
(87) Numéro de publication internationale: WO 2018/178569

(56) Documents cités:
- US-A1- 2005 119 586
- FEKR ATENA ROSHAN ET AL: "Respiration Disorders Classification With Informative Features for m-Health Applications", IEEE JOURNAL OF BIOMEDICAL AND HEALTH INFORMATICS, IEEE, PISCATAWAY, NJ, USA, vol. 20, no. 3, 1 May 2016 (2016-05-01), pages 733 - 747, XP011609715, ISSN: 2168-2194, [retrieved on 20160509], DOI: 10.1109/JBHI.2015.2458965
- FERHAT ATTAL ET AL: "Physical Human Activity Recognition Using Wearable Sensors", SENSORS, vol. 15, no. 12, 11 December 2015 (2015-12-11), pages 31314 - 31338, XP055397645, DOI: 10.3390/s151229858
- MORILLO D S ET AL: "An Accelerometer-Based Device for Sleep Apnea Screening", IEEE TRANSACTIONS ON INFORMATION TECHNOLOGY IN BIOMEDICINE, IEEE SERVICE CENTER, LOS ALAMITOS, CA, US, vol. 14, no. 2, 1 March 2010 (2010-03-01), pages 491 - 499, XP011345650, ISSN: 1089-7771, DOI: 10.1109/TITB.2009.2027231

## Description

### DOMAINE DE L'INVENTION

La présente invention concerne le domaine du bien-être, en particulier la dynamique cardio-respiratoire pendant le sommeil.

Elle est applicable au grand public, indépendamment de toute structure médicale, par exemple lorsqu'une personne a besoin de mieux comprendre la structuration de son sommeil et les évènements apparaissant pendant son sommeil.

La présente invention permet en effet une analyse de la physiologie du sommeil, et trouve notamment une application dans le recueil et l'analyse de données pouvant être utiles ultérieurement à l'établissement d'un diagnostic relatif à la qualité du sommeil. Bien entendu, l'invention n'est pas une méthode de diagnostic dans la mesure où aucun tableau clinique n'est associé à la mise en oeuvre de celle-ci.

Les méthodes traditionnelles d'estimation de la qualité du sommeil sont souvent un compromis entre un coût de conception/fabrication et la complexité de mise en oeuvre d'un dispositif mettant en pratique ladite méthode, et la robustesse de l'analyse et donc la pertinence de l'évaluation.

Il est connu le document EP0371424. Dans ce document il est prévu un ensemble de capteurs (électrodes cardiaques, microphone) permettant d'équiper un individu et d'enregistrer une pluralité de signaux tels que les bruits de ronflement et de respiration, ainsi que les battements cardiaques pendant son sommeil afin de déterminer, grâce à un calcul d'indexes, une éventuelle apnée obstructive du sommeil.

Il est connu le document EP2621336. Dans ce document il est prévu d'enregistrer les efforts respiratoires à partir d'un capteur de pression d'un individu éveillé et de déterminer un ensemble de paramètres afin de diagnostiquer une éventuelle apnée obstructive du sommeil sur la base d'un algorithme basé sur l'exhalaison et la comparaison des paramètres à un ensemble de bandes de fréquences.

Il est connu le document US9545227. Dans ce document il est prévu de déterminer le niveau de risque d'apnée du sommeil chez un individu. Pour cela, la méthode se base sur la détection d'un paramètre physiologique converti en flux de données prétraité dont sont extraites des caractéristiques. A partir de ces caractéristiques et d'informations issues de l'individu, un vecteur est construit afin de procéder à la détermination du niveau de risque par comparaison avec un modèle construit à partir d'apprentissage automatique.

Enfin, il est également connu le document WO2008037820. Dans ce document il est prévu un système de captation et d'analyse de signaux cardio-respiratoires caractérisé en ce qu'un seul et unique accéléromètre est utilisé pour capter l'ensemble des signaux physiologiques recherchés (signaux cardiaques, respiratoires et de ronflement).

Ainsi, on trouve d'un côté des méthodes se basant par exemple sur l'analyse de signaux captés par une centrale inertielle d'un objet communicant posé sur le lit d'un individu en train de dormir.

Cependant, outre le fait que les composants d'un objet communicant ne sont pas toujours de qualité suffisante pour obtenir un signal optimal pour une analyse, le nombre de paramètres physiologiques mesurés est également faible. Seule l'absence de matériel spécifique à la captation de signaux physiologiques rend la solution peu coûteuse pour l'utilisateur, ainsi que le développement peu complexe pour le concepteur.

D'un autre côté, on retrouve des solutions médicales ou paramédicales, par exemple de polygraphie ventilatoire ou polysomnographie (PSG), se basant sur une approche multimodale et multi-capteurs. Souvent, ces méthodes tentent d'analyser, via une lecture *in extenso* d'un spécialiste, la dynamique cardio-respiratoire de l'individu (entre autres) pour déterminer un état du sommeil ou identifier des événements typiques, représentatifs d'un trouble du sommeil. Plus particulièrement, le spécialiste cherche à déterminer les événements respiratoires de type apnées et hypopnées obstructives ou centrales. Ces données sont usuellement synthétisées sous la forme d'un indice unique appelé IAH pour Index Apnées - Hypopnées et qui correspond à la somme des évènements détectés / unité de temps, en l'espèce l'heure.

Les capteurs impliqués sont typiquement de plusieurs types (ECG, pléthysmographie respiratoire, thermistance naso-bucale ...), augmentant mécaniquement le coût de fabrication et de conception des solutions. Les signaux obtenus sont de plus de qualité variable ne permettant pas toujours une analyse robuste car l'individu peut bouger pendant la nuit.

En outre, cette multiplicité des capteurs a pour conséquence une complexité d'utilisation pour l'utilisateur ainsi qu'une acceptabilité moindre puisque celui-ci doit porter l'ensemble des capteurs, sur son torse, ses doigts, son crâne, etc. ce qui *in fine* influe négativement sur la qualité du sommeil.

Dans ce contexte, le demandeur a souhaité résoudre ces problèmes de coûts et de complexité de mise en œuvre associés aux solutions existantes, tout en conservant une très bonne robustesse d'analyse.

En ce sens, la solution proposée ici consiste en une approche basée par exemple sur un couple d'accéléromètres embarqués au sein d'un même dispositif matériel disposé en un endroit unique de l'individu au niveau du tronc de l'individu, ce qui rend l'invention pratique, compacte et facile à mettre en œuvre. On peut en outre incorporer, dans le but d'améliorer la robustesse des mesures explicitées ci-après, un ensemble d'au moins un capteur additionnel parmi un capteur de photopléthysmographie (PPG), qui permet une mesure optique indirecte de l'oxygénation du sang et les battements cardiaques ; un microphone et un gyroscope, permettant de suivre la position angulaire dudit dispositif.

Au-delà des avancées technologiques proposées par la présente invention, celle-ci répond à un double besoin de santé publique et de pratique médicale : certaines populations ignorent leurs troubles du sommeil et par conséquent ne sont pas orientées dans le parcours santé. La présente invention peut permettre de détecter ces populations et peut permettre en ce sens de répondre à un besoin de santé publique, puisqu'elle permet un dépistage, éventuellement précoce, sur ces populations. De plus les techniques standard (PSG) nécessitent une expertise compliquée à obtenir et pratiquer et ainsi limitent le nombre d'experts disponibles. La présente invention permet de favoriser la diffusion d'un outil précis pour l'évaluation de la santé cardio-respiratoire du sommeil auprès de praticiens non spécialistes.

FEKR ATENA ROSHAN ET AL: "Respiration Disorders Classification With Informative Features for m-Health Applications",IEEE JOURNAL OF BIOMEDICAL AND HEALTH INFORMATICS, IEEE, PISCATAWAY, NJ, USA, vol. 20, no. 3, 1 mai 2016 décrivent un système qui utilise des capteurs de mouvement pour détecter les changements dans le diamètre antérieur-postérieur de la paroi thoracique pendant la fonction respiratoire ainsi que pour extraire les caractéristiques respiratoires informatives à utiliser pour la classification des troubles respiratoires.

### RESUME DE L'INVENTION

Plus précisément, l'invention concerne un système de détermination d'un ensemble d'au moins un descripteur cardio-respiratoire d'un individu pendant son sommeil selon les revendications 1 à 12.

On peut prévoir que le dispositif de filtrage des données est configuré pour :
∘ extraire une plage basses fréquences, une plage moyennes fréquences et une plage hautes fréquences des données issues du premier ensemble d'au moins un accéléromètre, et
∘ extraire au moins une plage basses fréquences des données issues du deuxième ensemble d'au moins un accéléromètre.

On peut prévoir en outre :
- des moyens de détermination de la position dudit individu au cours de son sommeil, par les données issues de l'un au moins parmi ledit premier ensemble d'au moins un accéléromètre et ledit deuxième ensemble d'au moins un accéléromètre.

On peut prévoir en outre l'un au moins des capteurs parmi :
- un capteur de photopléthysmographie (PPG),
- un microphone et
- un ensemble d'au moins un capteur de mesure de position angulaire, synchronisé avec le premier ensemble d'au moins un accéléromètre et configuré pour être placé en position thoracique dudit individu ;

- le dispositif de filtrage des données étant en outre configuré pour :
   - coupler les données d'un ensemble d'au moins un capteur de mesure de position angulaire avec les données de l'un au moins parmi le premier ensemble d'au moins un accéléromètre et le deuxième ensemble d'au moins un accéléromètre, pour l'extraction de caractéristiques de position de l'utilisateur ou de variations vitesse angulaire respiratoires atypiques ; et/ou
   - moyenner les données issues du capteur de photopléthysmographie optique.

On peut prévoir que le calculateur est configuré pour déterminer un ensemble d'au moins un descripteur cardio-respiratoire de l'individu parmi :
- un index d'apnées-hypopnées (IAH),
- un premier ensemble d'efforts respiratoires dans les données issues du premier ensemble d'au moins un accéléromètre,
- un deuxième ensemble d'efforts respiratoires dans les données issues du deuxième ensemble d'au moins un accéléromètre,
- un indice de désynchronisation thorax-abdomen, par comparaison synchronisée des efforts respiratoires premier ensemble d'efforts respiratoires et des efforts respiratoires deuxième ensemble d'efforts respiratoires,
- la variabilité de la fréquence cardiaque (HRV),
- le profil d'oxymétrie ou saturation en oxygène,
- l'entropie respiratoire par unité de temps,
- le pourcentage de ronflement par unité de temps ; et
- un profil nocturne de la saturation en oxygène.

Par exemple, le pourcentage de ronflement par unité de temps est déterminé au moins en partie en fonction des données issues de l'un au moins parmi le premier ensemble d'au moins un accéléromètre et le microphone.

Par exemple, le profil nocturne de la saturation en oxygène est déterminé au moins en partie en fonction des données issues du capteur PPG thoracique.

On peut prévoir que le calculateur est configuré pour sélectionner un ensemble de caractéristiques parmi l'ensemble des caractéristiques déterminées, par exemple par un algorithme de type Sequential Feature Selection (SFS) ou Fast Correlation-Based Filter (FCBF), préalablement à la comparaison.

On peut prévoir que le calculateur est configuré pour calculer l'un au moins parmi :
- la corrélation temporelle entre au moins deux événements déterminés sur une même plage de temps ou sur plusieurs plages de temps ;
- un ensemble de couples (événement / position),
- un ensemble de couples (événement / caractéristique),
- un ensemble de triplets (événement / caractéristique / position),
- un ensemble de quadruplets (événement / événement / caractéristique / position).

On peut prévoir que le calculateur est configuré pour déterminer une chaine d'événements qui, pendant une fenêtre temporelle prédéterminée, a abouti à un descripteur cardio-respiratoire de valeur anormale.

On peut prévoir en outre des moyens d'activation / désactivation des accéléromètres et capteurs optionnels.

On peut prévoir que la mémoire pour enregistrer les données issues des accéléromètres et des capteurs optionnels et la mémoire pour enregistrer le modèle de référence sont les mêmes ;
ladite mémoire étant :
- soit déportée,
- soit en connexion informatique filaire avec le premier ensemble d'au moins un accéléromètre, le deuxième ensemble d'au moins un accéléromètre ainsi que les capteurs optionnels.

D'autres caractéristiques et avantages de la présente invention apparaîtront plus clairement à la lecture de la description suivante donnée à titre d'exemple illustratif et non limitatif et faite en référence aux figures annexées.

### DESCRIPTIF DES DESSINS

la figure 1 illustre un mode de réalisation du système selon l'invention,
la figure 2A illustre un mode de positionnement des accéléromètres et des capteurs optionnels selon l'invention,
la figure 2B illustre un autre mode de positionnement des accéléromètres et des capteurs optionnels selon l'invention, et
la figure 3 illustre les plages de fréquences au sens de l'invention.

### DESCRIPTION DETAILLEE

Par convention de langage, on entend par « individu » « sujet » ou « utilisateur » une personne physique utilisatrice d'un dispositif de mesure selon l'invention.

Par « sommeil » on entend une période de temps, éventuellement variable, pendant laquelle le dispositif de mesure selon l'invention est actif ; l'individu étant normalement endormi, éventuellement de façon discontinue, pendant une partie au moins de cette période de temps.

### Dispositif de mesure

On prévoit un dispositif de mesure unique et compact comportant au moins deux accéléromètres.

En particulier, on prévoit un premier ensemble d'au moins un accéléromètre, destiné à être placé en position thoracique de l'individu, de préférence en position xiphoïde, ce qui permet de mesurer l'activité mécanique cardiaque, les efforts ventilatoires thoraciques et l'activité de ronflement.

On prévoit également un deuxième ensemble d'au moins un accéléromètre, destiné à être placé en position abdominale dudit individu, ce qui permet de mesurer les efforts ventilatoires de l'abdomen.

Chaque ensemble d'au moins un accéléromètre peut comprendre une pluralité d'accéléromètres par exemple pour des raisons de redondance et robustesse.

En l'espèce, des essais réalisés par le demandeur ont prouvé qu'un seul accéléromètre par ensemble peut suffire.

On peut prévoir également en outre l'ajout d'autres capteurs optionnels, par exemple au moins l'un parmi un capteur de Photopléthysmographie, ci-après capteur PPG, et un microphone, placés en l'espèce en position thoracique au niveau xiphoïde. On peut aussi prévoir un ensemble d'au moins un capteur de mesure de position angulaire, en l'espèce un gyroscope, en combinaison avec un ensemble d'au moins un accéléromètre.

Par conséquent, le dispositif de mesure illustré ici comprend notamment deux accéléromètres, et, par concision, le premier ensemble d'au moins un accéléromètre est appelé premier accéléromètre 110, et le deuxième ensemble d'au moins un accéléromètre est appelé deuxième accéléromètre 120.

Chaque capteur (par exemple accéléromètre) émet un signal respectif comprenant un ensemble de données. Par concision, on entend donc indistinctement « signal » et « données ».

De préférence, on prévoit que le dispositif de mesure comprend des moyens d'activation / désactivation des capteurs, par exemple sous forme d'interrupteur de commande d'activation, analogique ou numérique.

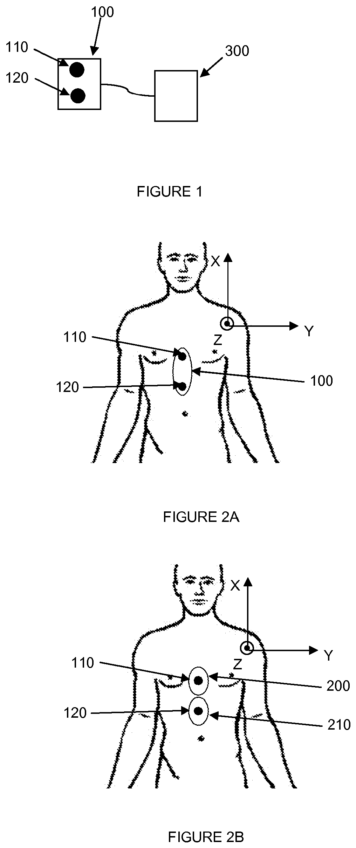

Dans une première variante, illustrée figure 2A, on prévoit que le premier accéléromètre, le deuxième accéléromètre et les capteurs optionnels sont solidaires d'un unique support 100.

Dans une seconde variante, illustrée figure 2B, on prévoit que le premier accéléromètre et le deuxième accéléromètre sont solidaires d'un support respectif 200, 210. Les capteurs optionnels peuvent être solidaires du support du premier ensemble d'au moins un accéléromètre.

Quelle que soit la variante, chaque support comprend une face supérieure et une face inférieure. La face supérieure supporte au moins un accéléromètre et la face inférieure est typiquement enduite d'adhésif (colle), de préférence repositionnable. L'avantage de l'aspect repositionnable est que l'individu peut retirer le dispositif de mesure au réveil et réutiliser le même dispositif de mesure la nuit suivante, et ce sur plusieurs jours consécutifs. Le dispositif est par exemple alimenté par une batterie.

Le premier accéléromètre et le deuxième accéléromètre peuvent être identiques. En l'espèce, le premier accéléromètre et le deuxième accéléromètre sont des accéléromètres trois axes standards, en l'espèce avec X l'axe d'élongation de l'individu et l'axe Z qui est orthogonal au sujet (figure 2A, figure 2B).

Les données issues de chaque capteur sont enregistrées dans une mémoire informatique.

Dans une première variante, la mémoire est en connexion informatique filaire avec le premier et le deuxième accéléromètre ainsi que les capteurs optionnels. La mémoire peut par exemple être disposée sur le support unique ou l'un quelconque des supports des accéléromètres.

De préférence, on prévoit que la mémoire est en connexion informatique avec un port de communication d'entrée/sortie (I/O), par exemple un port USB, ce qui permet d'exporter les données enregistrées vers un dispositif de traitement des données comprenant un logiciel de traitement des données, typiquement tout objet communicant, c'est-à-dire un dispositif électronique comprenant des moyens de communication avec ou sans fil, un calculateur 300 (processeur) et de préférence un écran d'affichage, par exemple un ordinateur personnel, un *smartphone,* une tablette tactile etc.

On peut aussi prévoir que la mémoire est amovible, par exemple sous forme de support de donnée de type carte mémoire à connexion USB, connectable au premier accéléromètre et au deuxième accéléromètre pour l'enregistrement des données issues de ceux-ci, et connectable ensuite à un ordinateur équipé d'un logiciel de filtrage permettant de filtrer lesdites données.

Dans une deuxième variante, la mémoire est déportée, c'est-à-dire sans connexion filaire entre ladite mémoire et les capteurs. Par exemple, la mémoire est déportée dans le dispositif de traitement des données.

Dans ce cas, on prévoit avantageusement que le dispositif de mesure comprend des moyens de communication avec ledit dispositif de traitement des données, que ce soit une communication filaire ou sans fil.

La mémoire peut donc être embarquée ou déportée, par sur un serveur, en particulier d'un système informatique en nuage.

Les accéléromètres et les capteurs optionnels sont montés avantageusement sur une carte électronique, comportant une batterie, un calculateur, par exemple un processeur (microcontrôleur) et une unité de stockage pouvant stocker les données dans l'optique d'un traitement embarqué. Alternativement, chaque accéléromètre est monté sur une carte électronique indépendante, chacune disposant d'une pile d'alimentation, d'un calculateur (processeur) et d'une unité de stockage. On prévoit que les capteurs optionnels sont solidaires de la carte électronique du premier ensemble d'au moins un accéléromètre La synchronisation des signaux est faite par construction par le microcontrôleur.

De préférence, on prévoit également des moyens de traitement des signaux issus des capteurs.

En l'espèce, les moyens de traitement des signaux comprennent des moyens de filtrage, configurés pour filtrer le signal de sortie du premier accéléromètre, et configurés pour filtrer le signal de sortie du deuxième accéléromètre.

De manière similaire à la mémoire, les moyens de filtrage peuvent être intégrés au dispositif de mesure, ou déportés sur le dispositif de traitement des données ou encore sur un serveur en communication avec le dispositif de traitement des données.

Dans ce cas, on prévoit avantageusement que le dispositif de mesure comprend des moyens de communication avec lesdits moyens de traitement des signaux, que ce soit une communication filaire ou sans fil.

Le capteur de Photopléthysmographie comprend un émetteur et un récepteur optique permettant de mesurer la saturation en oxygène du sang au niveau thoracique par la différence d'absorption entre Hémoglobine et Oxyhémoglobine entre la lumière rouge et infrarouge.

Chaque signal généré par les accéléromètres est traité pour le rendre exploitable, puis analysé sur plusieurs bandes de fréquences pour en extraire différentes valeurs de paramètres physiologiques susceptibles d'indiquer la survenue d'événements typiques de troubles respiratoires du sommeil donc affectant la qualité du sommeil, comme décrit ci-après.

### Traitement des données

On prévoit de traiter les données issues des accéléromètres de la manière suivante.

Le dispositif de mesure est installé sur l'individu, de préférence par celui-ci, avant le coucher, pour un fonctionnement en continu.

### Données brutes

Les données issues du premier accéléromètre et du deuxième accéléromètre sont appelées données brutes.

Les données brutes sont filtrées comme décrit ultérieurement, soit après leur enregistrement dans la mémoire, soit à la volée.

A titre d'exemple non limitatif, les données brutes fournies par les accéléromètres sont préférentiellement échantillonnées à une fréquence de 10 KHz.

A titre d'exemple non limitatif, les données brutes fournies par le capteur PPG sont préférentiellement échantillonnées à une fréquence de 25 Hz.

Avantageusement, l'acquisition se fait en continu sur toute la durée du sommeil.

### Données filtrées

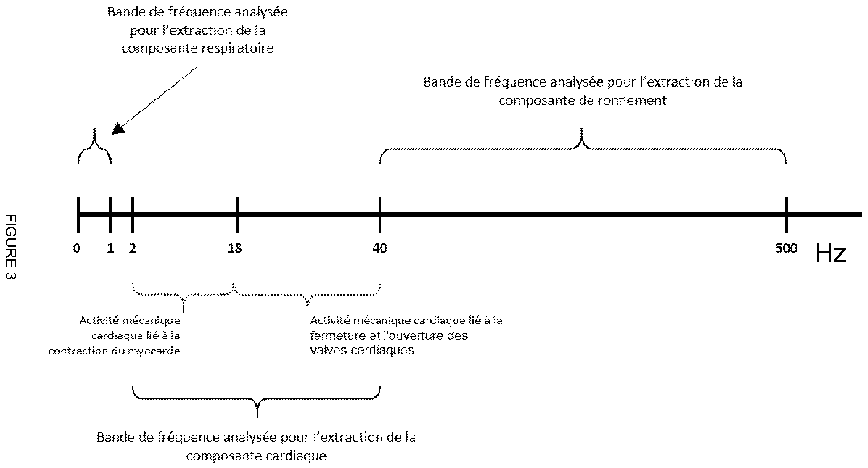

Les accéléromètres ont une bande passante fréquentielle préférentiellement comprise dans la gamme de 0 à 500 Hz, illustrée figure 3.

Typiquement, la gamme de fréquences ci-dessus peut être subdivisée selon les plages et la correspondance suivante :
- la plage de 0 à 1 Hz, ci-après plage « basses fréquences », correspond à une activité respiratoire de l'individu,
- la plage de 2 à 40 Hz, ci-après plage « moyennes fréquences », correspond à une activité cardiaque de l'individu, elle peut être subdivisée en :
   ∘ une sous-plage de 2 à 18 Hz qui correspond à une activité mécanique cardiaque liée à la contraction du myocarde de l'individu, et
   ∘ une sous-plage de 18 à 40 Hz qui correspond à une activité mécanique cardiaque liée à la fermeture et à l'ouverture des valves cardiaques de l'individu, et
- la plage de 40 à 500 Hz et au-delà, ci-après plage « hautes fréquences », correspond à une activité de ronflement de l'individu.

La plage basses fréquences est la composante respiratoire du signal d'un accéléromètre, elle est induite par les mouvements de la cage thoracique de l'individu lors de sa respiration.

La plage moyennes fréquences est la composante cardiaque du signal d'un accéléromètre, elle est induite par l'activité cardiaque de l'individu.

La plage hautes fréquences est la composante de ronflement du signal d'un accéléromètre, elle est induite par les vibrations dues aux ronflements dudit individu.

Chacune des activités de l'individu (respiratoire, cardiaque et ronflement) font en effet vibrer le ou les accéléromètres selon une plage spécifique et exclusive de fréquences.

On prévoit donc avantageusement une étape de filtrage consistant à filtrer les signaux issus des accéléromètres pour extraire desdits signaux l'une au moins des composantes, ou indistinctement bandes ou plages, parmi la « basses fréquences », la « moyennes fréquences » et la « hautes fréquences ».

En l'espèce, on prévoit d'extraire :
- la composante respiratoire par tout filtre passe bas connu, et par exemple par analyse en composantes principales (ACP) ou par transformée de Fourier discrète ;
- la composante cardiaque par tout filtre passe bande connu, et par exemple par filtrage adaptatif, par extraction d'enveloppe, par apprentissage, par corrélation croisée, par partitionnement en k-moyennes, ou par analyse en composantes principales (ACP), et
- la composante de ronflement par tout filtre passe haut connu, et par exemple par analyse spectrale.

De préférence, on prévoit d'extraire la composante respiratoire, la composante cardiaque et la composante de ronflement des données brutes issues du premier accéléromètre, placé en position thoracique de l'individu.

De préférence, on prévoit d'extraire au moins la composante respiratoire des données brutes issues du deuxième accéléromètre placé en position abdominale de l'individu.

Avantageusement, avec une disposition d'accéléromètres trois axes X, Y et Z telle qu'illustrée en figure 2A et en figure 2B, avec XoZ le plan sagittal ou médian, XoY le plan coronal ou frontal et YoZ le plan axial ou transversal, on peut extraire :
- la composante respiratoire par une combinaison linéaire des 3 axes, en l'espèce sous forme de vecteur ;
- la composante cardiaque en n'utilisant que la composante selon l'axe Z du signal de chaque accéléromètre,
- la composante de ronflement par une combinaison linéaire des 3 axes, en l'espèce sous forme de vecteur.

On peut en outre prévoir avantageusement de pondérer la composante de ronflement extraite selon l'un au moins des axes X, Y et Z de manière adaptative, en fonction de la position de l'individu, la détermination de la position de l'individu étant décrite ultérieurement.

On peut aussi prévoir une étape de lissage des signaux après extraction des composantes dans chacune des bandes de fréquence, ce qui permet d'enlever les artéfacts.

Au niveau du capteur PPG, on calcule la différence d'absorption entre Hémoglobine et Oxyhémoglobine entre la lumière rouge et infrarouge. On considère ensuite un filtre moyenne avec un temps de moyennage intermédiaire permettant un compromis entre précision de la mesure et le rejet d'artefact. Ce temps est à titre d'exemple non limitatif de 1 seconde.

### Synchronisation

Les données (brutes ou filtrées) issues du deuxième accéléromètre sont synchronisées avec les données (brutes ou filtrées) issues du premier accéléromètre et les données des capteurs optionnels, par exemple par un calculateur (processeur) embarqué (microcontrôleur) ou un système d'acquisition (serveur).

Par exemple, on peut prévoir un système d'acquisition, en l'espèce un CPU ou un microcontrôleur, couplé à une horloge et un échantillonneur et un convertisseur analogiquenumérique.

Comme décrit ultérieurement, la position de l'individu peut être déterminée au cours de son sommeil. Cette position peut être synchronisée avec les caractéristiques (respiratoires, cardiaques, ronflements) déterminées, comme décrit ci-dessous.

### Détermination de caractéristiques

A partir des données issues des accéléromètres (brutes et de préférence filtrées) et le cas échéant en outre d'au moins l'un parmi un capteur PPG, un microphone, et un ensemble d'au moins un gyroscope, il est possible de déterminer, par bande de fréquence, un ensemble d'au moins une caractéristique représentative d'un trouble du sommeil ayant normalement eu lieu en phase de sommeil de l'individu, ainsi que le moment, c'est-à-dire l'instant ou la plage de temps, auquel ladite caractéristique a été extraite.

En effet, les troubles respiratoires du sommeil se traduisent par des conditions physiques ou physiologiques de l'individu : ronflements, retournement(s), agitation, positionnement de l'individu, respiration (ou absence de) thoracique, respiration (ou absence de) abdominale, etc. La convergence de ces évènements permet de pointer vers un état potentiellement pathologique.

Ces conditions physiques ou physiologiques se traduisent à leur tour par des caractéristiques mesurables, qui sont extraites des signaux issus des accéléromètres et le cas échéant des capteurs optionnels.

La plage « basses fréquences » des accéléromètres est représentative des efforts respiratoires, qui comprennent par exemple l'inspiration et l'expiration de l'individu.

On peut en extraire des caractéristiques telles que :
- la fréquence respiratoire, c'est-à-dire tant la fréquence respiratoire thoracique que la fréquence respiratoire abdominale,
- l'amplitude respiratoire,
- l'entropie respiratoire,
- etc.

La plage « moyennes fréquences » est représentative des contractions du cœur de l'individu.

On peut en extraire des caractéristiques telles que :
- la fréquence cardiaque instantanée, au moins grâce au premier accéléromètre ,
- le temps entre deux battements successifs,
- etc.

La plage « hautes fréquences » est représentative des ronflements de l'individu.

On peut en extraire des caractéristiques telles que :
- les moments ou la fréquence de ronflement de l'individu, au moins grâce au premier accéléromètre,
- l'analyse spectrale de ronflement (puissance, amplitude, ...)
- etc.

Pour les capteurs optionnels, on prévoit d'extraire des caractéristiques représentant la saturation en oxygène de l'individu au niveau thoracique grâce au capteur PPG.

A partir de l'un au moins parmi le premier accéléromètre, le deuxième accéléromètre et au moins un capteur de position angulaire, typiquement un gyromètre (indistinctement gyroscope), on peut prévoir d'extraire des caractéristiques de position angulaire permettant préférentiellement une optimisation algorithmique pour :
- Le calcul de la position de l'utilisateur,
- La détection, en association avec au moins un ensemble d'accéléromètres, d'éventuels schémas, ou patterns par anglicisme, respiratoires anormaux (variation angulaire atypique lors de la reprise respiratoire post-apnée obstructives par exemple).

Les plages « basses fréquences », « moyennes fréquences » et « hautes fréquences » peuvent être traitées en série ou en parallèle.

On peut aussi prévoir en outre de sélectionner un ensemble de caractéristiques parmi l'ensemble de caractéristiques déterminées, en particulier par un algorithme de type Sequential Feature Selection (SFS) ou Fast Correlation-Based Filter (FCBF), ce qui permet d'optimiser les calculs.

### Troubles du sommeil - événements

Les troubles du sommeil de l'individu se caractérisent notamment par l'un au moins des syndromes parmi :
- le syndrome d'apnées obstructive du sommeil (SAOS), et
- le syndrome d'apnées du sommeil centrales (SASC).

Ces syndromes correspondent typiquement à l'apparition de l'un au moins des événements (physiologiques) suivants pendant le sommeil de l'individu :
- une apnée obstructive,
- une apnée centrale,
- une apnée mixte,
- une hypopnée,
- une hyperventilation et
- une hypoventilation.

Les événements ci-dessus peuvent être caractérisés par une distribution spécifique dans le temps des caractéristiques déterminées.

D'un point de vue physiologique, dans la plupart des cas de SAOS, l'air s'arrête de circuler en direction des, et depuis les, poumons à cause d'un blocage dans les voies aériennes supérieures, dans le nez ou dans la gorge.

A la différence du SAOS, le SASC peut survenir même lorsque les voies aériennes sont dégagées. L'ACS signifie que l'individu arrête de respirer pendant un nombre prédéterminé de secondes consécutives au cours de son sommeil, en l'espèce au moins 10 secondes consécutives.

L'hypopnée, ou respiration superficielle, quant à elle, est une diminution du débit respiratoire, dont les bornes sont plus ou moins bien définies, pendant un nombre prédéterminé de secondes consécutives au cours de son sommeil. En l'espèce, on considère qu'une diminution d'au moins 50 % du débit respiratoire pendant 10 secondes définit une hypopnée. On peut prévoir que l'hypopnée est en outre définie avec une diminution de 3 % à 4 % de la saturation (oxygène dans le sang).

En pratique, l'apnée/hypopnée peut donc être caractérisée par des arrêts répétés de la respiration ou une respiration faible pendant de courtes périodes de temps durant le sommeil. En termes anatomiques, il y a un effondrement intermittent des voies aériennes supérieures qui se traduit possiblement par une réduction des concentrations sanguines en oxygène pendant le sommeil. Ainsi, une personne endormie devient incapable de respirer normalement et le plus souvent se réveille avec chaque effondrement.

En réalité, ces définitions sont relatives et les bornes sont non clairement établies du fait de définitions différentes selon les auteurs et de l'absence d'harmonisation. Autrement dit, deux experts différents face aux mêmes résultats peuvent émettre deux interprétations différentes, y compris par exemple par la prise en compte ou non de facteurs environnants à l'individu (facteurs de risques, antécédents médicaux, etc.) dans leur diagnostic.

La présente invention permet de s'extraire de cette contrainte.

### Modèle de référence

On prévoit un modèle de référence. En l'espèce, le modèle de référence comprend un premier groupe de données physiologiques relatives à des individus reconnus comme étant atteints de troubles du sommeil, par exemple un ensemble de polysomnographies.

Le modèle de référence comprend également avantageusement un deuxième groupe de données physiologiques, relatives à des individus reconnus comme non atteints de troubles du sommeil, par exemple un ensemble de polysomnographies.

En particulier, on prévoit qu'au moins le premier groupe de données physiologiques comprend une correspondance entre un ensemble de caractéristiques distribuées dans le temps et un événement donné (apnée obstructive, apnée centrale, apnée mixte, hypopnée, hyperventilation ou hypoventilation).

Il est alors possible de comparer les caractéristiques (déterminées ou sélectionnées) avec le modèle de référence, en l'espèce au moins avec les données du premier groupe, pour en déduire le ou les événements correspondants probables auxquels l'individu a fait face pendant la période de temps considérée, ce qui permet de détecter un ensemble d'événements potentiellement marqueurs de troubles du sommeil et d'effectuer une forme de dépistage de troubles du sommeil au lieu d'un diagnostic qui vise quant à lui à identifier la nature et la cause de l'affection dont un patient est atteint.

De préférence, l'étape de comparaison est mise en œuvre par apprentissage automatique (*machine learning* par anglicisme), ce qui consiste à construire un modèle décisionnel, par exemple par machines à vecteurs de support (support vector machine ou SVM par anglicisme), par approche gaussienne, par approche Bayésienne, etc. notamment pour la détection et discrimination des événements respiratoires anormaux.

La comparaison est faite par exemple sur les caractéristiques extraites suivantes :
- respiratoires (fréquence, amplitude, index de synchronisation thoraco-abdominal),
- cardiaque (fréquence, HRV),
- ronflements (composition spectrales).
- saturation en oxygène thoracique (capteur PPG)

L'apprentissage au niveau du traitement des signaux issus des premier et deuxième accéléromètres permet de rendre plus robuste les caractéristiques extraites et donc d'améliorer les performances de l'algorithme de comparaison. Par exemple, le traitement du signal issu de la plage « moyennes fréquences » doit résulter en une fréquence cardiaque suffisamment précise.

En l'espèce l'étape de comparaison est mise en oeuvre par apprentissage supervisé par le modèle de référence.

### Classification et détermination de descripteurs cardio-respiratoires

A partir des caractéristiques déterminées, représentatives d'un trouble potentiel du sommeil, on peut déterminer un ensemble d'au moins un descripteur cardio-respiratoire de l'individu, chaque descripteur étant représentatif de la survenue d'événement(s) représentatif(s) de trouble(s) du sommeil pendant le sommeil de l'individu ; c'est-à-dire le type de trouble (apnée, hypopnée, hyper ou hypoventilation), le moment où l'événement survient, et le nombre de fois où ces événements ont lieu, ainsi que leur fréquence s'ils sont réguliers.

Un descripteur cardio-respiratoire typique est par exemple l'IAH, calculé à partir du nombre et du type d'événements respiratoires détectés par heure de sommeil (apnées obstructives, centrales ou mixtes/ hypopnées obstructives ou centrales).

Un autre descripteur cardio-respiratoire est l'indice de désynchronisation thorax-abdomen par unité de temps. Celui-ci permet d'affiner la nature centrale ou obstructive des apnées.

Dans ce cas, on peut comparer les efforts respiratoires issus des données filtrées du deuxième accéléromètre (abdominal) et les efforts respiratoires issus des données filtrées du premier accéléromètre (thoracique). L'indice de désynchronisation thorax-abdomen est calculé par une estimation du déphasage entre les signaux issus du premier accéléromètre et les signaux issus du deuxième accéléromètre. On peut alors estimer le retard de l'un par rapport à l'autre par comparaison de leurs passages respectifs par 0, puis par le calcul d'une transformée en ondelettes pour comparaison de la phase spectrale.

Un autre descripteur cardio-respiratoire est la variabilité de la fréquence cardiaque (VFC ou HRV pour Heart Rate Variability en anglais), qui est la fluctuation de la durée des intervalles de temps entre deux battements consécutifs du cœur.

Elle résulte essentiellement de régulations extrinsèques et détermine la fréquence cardiaque. Alors que la fréquence cardiaque peut être quasi-stable sur une période d'intégration temporelle donnée, le temps entre deux battements cardiaques peut être très différent et sa valeur informative est plus importante.

Dans ce cas, on prévoit que l'extraction de la composante cardiaque comprend en outre une étape de détermination de la variabilité du rythme cardiaque, en l'espèce par analyse temporelle et spectrale du signal du premier accéléromètre dans la plage « moyennes fréquences ».

Un autre descripteur cardio-respiratoire est l'index de désaturation en oxygène sur la nuit de mesure. Il rend compte du nombre de désaturation supérieur à 3% sur la nuit, de la plus grande désaturation en oxygène sur la nuit et du profil moyen sur la nuit.

Un autre descripteur cardio-respiratoire est l'entropie respiratoire par unité de temps.

Un autre descripteur cardio-respiratoire est le pourcentage de ronflement par unité de temps.

Avantageusement, au moins l'un des descripteurs cardio-respiratoire déterminé est couplé à la position de l'individu. On peut en effet associer la survenue d'événements marqueurs de troubles du sommeil, déterminés par l'étape de comparaison, à la position de l'individu à ce moment-là ou dans une fenêtre de temps précédente prédéterminée, ce qui permet de déterminer des descripteurs cardio-respiratoires pour lesquels les données physiologiques de l'individu sont couplées à sa posture de manière synchronisée.

On peut ainsi déterminer par exemple :
- un ensemble de couples ronflement / position ; HRV / position ; ronflement / fréquence cardiaque ; HRV / fréquence cardiaque ; etc.
- un ensemble de triplets ronflement / fréquence cardiaque / position ; etc.
- un ensemble de quadruplets ronflement / fréquence cardiaque / HRV / position ; etc.

On peut ainsi plus facilement déterminer la chaine d'événements qui, pendant une fenêtre temporelle prédéterminée, a abouti à une caractéristique cardio-respiratoire anormale, par exemple une augmentation de la fréquence cardiaque, une augmentation de l'amplitude des ronflements, etc. Pour déterminer si une caractéristique cardio-respiratoire est « anormale », on prévoit typiquement de comparer la valeur de ladite caractéristique avec une valeur de référence, de préférence comprise dans le modèle de référence.

En plus des événements déterminés ou sélectionnés, le modèle décisionnel peut utiliser en entrée d'autres informations relatives à l'individu, incluant mais non limitées à : son âge, son poids, sa taille, la circonférence de son cou, ses antécédents médicaux, ses habitudes alimentaires, sa consommation de produits reconnus comme susceptibles de perturber le sommeil (alcool et cigarettes notamment).

On peut aussi avantageusement prévoir de calculer la corrélation temporelle entre au moins deux événements physiologiques de l'individu sur une même plage de temps (simultanéité des événements) ou sur plusieurs plages de temps (diachronisme des événements, par exemple un retournement sur un côté qui induit un ronflement).

### Détermination de la position

On prévoit ensuite de déterminer la position de l'individu au cours de son sommeil par quantification des variations d'accélération sur les différents axes. Typiquement, les signaux issus de l'un au moins des accéléromètres et optionnellement d'un ensemble d'au moins un gyroscope permettent de déterminer si l'individu est dans l'une des positions parmi :
- allongé sur le dos,
- allongé sur le ventre,
- allongé sur le côté droit,
- allongé sur le côté gauche,
- assis ou semi-assis, et
- debout.

Grâce à l'invention, il devient alors possible d'effectuer une analyse statistique par corrélation croisée, ce qui permet de mieux définir les conditions dans lesquelles l'individu présente des troubles du sommeil, par exemple une accélération cardiaque quand celui-ci ronfle sur le côté gauche.

Les données issues des premier et deuxième accéléromètres et le cas échéant des capteurs additionnels optionnels sont analysées (en temps réel ou a posteriori) par une succession de fenêtres glissantes dont la durée est prédéterminée (par exemple quelques secondes) pendant une période totale prédéterminée (par exemple quelques minutes) et les caractéristiques déterminées pendant cette période totale prédéterminée sont comparées aux données du modèle de référence.

Les données issues des premier et deuxième accéléromètres, du capteur PPG, du microphone ou la position de l'individu, peuvent aussi servir de discriminant aux événements déterminés ou sélectionnés. Par exemple une accélération cardiaque associée à une accélération verticale indique que l'individu est certainement en train de se lever, donc éveillé, et les événements correspondants peuvent alors être discriminés de l'étape de comparaison au modèle de référence.

Grâce à la présente invention, il est possible d'obtenir une dynamique des mesures sur plusieurs nuits alors que l'art antérieur ne permet qu'une nuit unique.

Le temps d'analyse selon les solutions de l'art antérieur est généralement de l'ordre de une à deux heures de travail pour analyser une nuit d'un individu. Au contraire selon l'invention le temps de calcul est celui d'un calculateur (processeur), soit quelques secondes. En outre le calcul est automatique, c'est-à-dire indépendant de toute intervention humaine, en particulier de spécialiste médical.

La présente invention permet la détermination d'événements corrélés et anormaux, comme le suivi de traitement des troubles du sommeil. Elle est non intrusive, ne nécessite aucun équipement traditionnel encombrant (casque, EEG, EMG, dispositif de mesure de l'oxygénation, de mesure du débit d'air, etc.) et peut ne nécessiter que deux accéléromètres, pour une finesse de mesure et d'analyse adaptée à la démarche de dépistage précoce dans la population générale ou pathologique.

## Revendications

1. Système de détermination d'un ensemble d'au moins un descripteur cardio-respiratoire d'un individu pendant son sommeil, comprenant :
- un dispositif de mesure comprenant :
∘ un premier ensemble d'au moins un accéléromètre, configuré pour être placé en position thoracique de l'individu ; et
∘ un deuxième ensemble d'au moins un accéléromètre, synchronisé avec le premier ensemble d'au moins un accéléromètre et configuré pour être placé en position abdominale dudit individu ; et
∘ optionnellement au moins l'un parmi un capteur de photopléthysmographie (PPG), un microphone et un ensemble d'au moins un gyroscope synchronisé avec le premier ensemble d'au moins un accéléromètre ;
- une mémoire pour enregistrer les données issues des accéléromètres et des capteurs optionnels ;
**caractérisé en ce qu'**il comprend en outre :
- un modèle de référence enregistré dans une mémoire, le modèle comprenant une correspondance entre un ensemble de caractéristiques distribuées dans le temps et un ensemble d'événements physiologiques donnés, chaque événement étant de préférence représentatif d'un trouble potentiel du sommeil ;
- un dispositif de filtrage des données issues des accéléromètres configurés pour extraire des plages basses fréquences, moyennes fréquences et hautes fréquences, et
- un calculateur configuré pour :
∘ déterminer, par bande de fréquences extraite, un ensemble d'au moins une caractéristique représentative d'un état cardio-respiratoire et physiologique, ainsi que le moment auquel la dite caractéristique a été extraite ;
∘ comparer la distribution temporelle dudit ensemble d'au moins une caractéristique déterminée avec la distribution temporelle de caractéristiques similaires issues dudit modèle de référence;
∘ en déduire un ensemble d'au moins un événement correspondant probable auquel ledit individu a fait face pendant une période de temps prédéterminée ;
o déterminer, sur la base de l'un au moins parmi lesdits événements, un ensemble d'au moins un descripteur cardio-respiratoire de l'individu.

2. Système selon la revendication 1, dans lequel le dispositif de filtrage des données est configuré pour :
∘ extraire une plage basses fréquences, une plage moyennes fréquences et une plage hautes fréquences des données issues du premier ensemble d'au moins un accéléromètre, et
∘ extraire au moins une plage basses fréquences des données issues du deuxième ensemble d'au moins un accéléromètre.

3. Système selon l'une quelconque des revendications précédentes, comprenant en outre
- des moyens de détermination de la position dudit individu au cours de son sommeil, par les données issues de l'un au moins parmi ledit premier ensemble d'au moins un accéléromètre et ledit deuxième ensemble d'au moins un accéléromètre.

4. Système selon l'une quelconque des revendications précédentes, comprenant en outre l'un au moins des capteurs parmi :
- un capteur de photopléthysmographie (PPG),
- un microphone et
- un ensemble d'au moins un capteur de mesure de position angulaire, synchronisé avec le premier ensemble d'au moins un accéléromètre et configuré pour être placé en position thoracique dudit individu ;
- le dispositif de filtrage des données étant en outre configuré pour :
- coupler les données d'un ensemble d'au moins un capteur de mesure de position angulaire avec les données de l'un au moins parmi le premier ensemble d'au moins un accéléromètre et le deuxième ensemble d'au moins un accéléromètre, pour l'extraction de caractéristiques de position de l'utilisateur ou de variations vitesse angulaire respiratoires atypiques ; et/ou
- moyenner les données issues du capteur de photopléthysmographie optique.

5. Système selon l'une quelconque des revendications précédentes, dans lequel le calculateur est configuré pour déterminer un ensemble d'au moins un descripteur cardio-respiratoire de l'individu parmi :
• un index d'apnées-hypopnées (IAH),
• un premier ensemble d'efforts respiratoires dans les données issues du premier ensemble d'au moins un accéléromètre,
• un deuxième ensemble d'efforts respiratoires dans les données issues du deuxième ensemble d'au moins un accéléromètre,
• un indice de désynchronisation thorax-abdomen, par comparaison synchronisée des efforts respiratoires premier ensemble d'efforts respiratoires et des efforts respiratoires deuxième ensemble d'efforts respiratoires,
• la variabilité de la fréquence cardiaque (HRV),
• le profil d'oxymétrie ou saturation en oxygène,
• l'entropie respiratoire par unité de temps,
• le pourcentage de ronflement par unité de temps ; et
• un profil nocturne de la saturation en oxygène.

6. Système selon l'une quelconque des revendications précédentes, dans lequel le calculateur est configuré pour sélectionner un ensemble de caractéristiques parmi l'ensemble des caractéristiques déterminées, par exemple par un algorithme de type Sequential Feature Selection (SFS) ou Fast Correlation-Based Filter (FCBF), préalablement à la comparaison.

7. Système selon l'une quelconque des revendications précédentes, dans lequel le calculateur est configuré pour calculer l'un au moins parmi :
- la corrélation temporelle entre au moins deux événements déterminés sur une même plage de temps ou sur plusieurs plages de temps ;
- un ensemble de couples (événement / position),
- un ensemble de couples (événement / caractéristique),
- un ensemble de triplets (événement / caractéristique / position),
- un ensemble de quadruplets (événement / événement / caractéristique / position).

8. Système selon l'une quelconque des revendications précédentes, dans lequel le calculateur est configuré pour déterminer une chaine d'événements qui, pendant une fenêtre temporelle prédéterminée, a abouti à un descripteur cardio-respiratoire de valeur anormale.

9. Système selon l'une quelconque des revendications précédentes, comprenant en outre des moyens d'activation / désactivation des accéléromètres et capteurs optionnels.

10. Système selon l'une quelconque des revendications précédentes, dans lequel la mémoire pour enregistrer les données issues des accéléromètres et des capteurs optionnels et la mémoire pour enregistrer le modèle de référence sont les mêmes ;
ladite mémoire étant :
- soit déportée,
- soit en connexion informatique filaire avec le premier ensemble d'au moins un accéléromètre, le deuxième ensemble d'au moins un accéléromètre ainsi que les capteurs optionnels.

11. Système selon l'une quelconque des revendications précédentes, comprenant :
- un filtre passe bas, configuré pour extraire une composante respiratoire ;
- filtre passe bande, configuré pour extraire une composante cardiaque ; et
- un filtre passe haut, configuré pour extraire une composante de ronflement.

12. Système selon la revendication 11, dans lequel :
- le filtre passe bas est configuré pour mettre en œuvre une analyse en composantes principales (ACP) ou par transformée de Fourier discrète ;
- le filtre passe bande est configuré pour mettre en œuvre un filtrage adaptatif, par extraction d'enveloppe, par apprentissage, par corrélation croisée, par partitionnement en k-moyennes, ou par analyse en composantes principales (ACP), et
- le filtre passe haut est configuré pour mettre en œuvre une analyse spectrale.

## Patentansprüche

1. System zur Bestimmung eines Satzes von mindestens einem Herz-Lungen-Deskriptor einer Person während ihres Schlafs, beinhaltend:
- eine Messvorrichtung, beinhaltend:
o einen ersten Satz von mindestens einem Beschleunigungsmesser, der dazu konfiguriert ist, in einer Thoraxposition der Person platziert zu werden; und
o einen zweiten Satz von mindestens einem Beschleunigungsmesser, der mit dem ersten Satz von mindestens einem Beschleunigungsmesser synchronisiert ist und dazu konfiguriert ist, in einer Abdomenposition der Person platziert zu werden; und
o optional mindestens eines von einem Photoplethysmographie(PPG)-Sensor, einem Mikrofon und einem Satz von mindestens einem Gyroskop, der mit dem ersten Satz von mindestens einem Beschleunigungsmesser synchronisiert ist;
- einen Speicher, um die aus den Beschleunigungsmessern und den optionalen Sensoren stammenden Daten aufzuzeichnen;
**dadurch gekennzeichnet, dass** es ferner Folgendes beinhaltet:
- ein Referenzmodell, das in einem Speicher aufgezeichnet ist, wobei das Modell eine Beziehung zwischen einem Satz von zeitlich verteilten Eigenschaften und einem Satz von gegebenen physiologischen Ereignissen beinhaltet, wobei jedes Ereignis vorzugsweise für eine potenzielle Störung des Schlafs repräsentativ ist;
- eine Vorrichtung zum Filtern der aus den Beschleunigungsmessern stammenden Daten, die dazu konfiguriert ist, Niederfrequenz-, Mittelfrequenz- und Hochfrequenzbereiche zu extrahieren, und
- eine Recheneinheit, die zu Folgendem konfiguriert ist:
o Bestimmen, pro extrahiertem Frequenzband, eines Satzes von mindestens einer Eigenschaft, die für einen Herz-Lungen- und physiologischen Zustand repräsentativ ist, sowie des Zeitpunkts, zu dem die Eigenschaft extrahiert wurde;
o Vergleichen der zeitlichen Verteilung des Satzes von mindestens einer bestimmten Eigenschaft mit der zeitlichen Verteilung von ähnlichen Eigenschaften, die aus dem Referenzmodell stammen;
o daraus Ableiten eines Satzes von mindestens einem möglichen entsprechenden Ereignis, dem die Person während eines vorbestimmten Zeitraums ausgesetzt war;
o Bestimmen, auf der Basis des mindestens einen der Ereignisse, eines Satzes von mindestens einem Herz-Lungen-Deskriptor der Person.

2. System nach Anspruch 1, wobei die Vorrichtung zum Filtern der Daten zu Folgendem konfiguriert ist:
o Extrahieren eines Niederfrequenzbereichs, eines Mittelfrequenzbereichs und eines Hochfrequenzbereichs der Daten, die aus dem ersten Satz von mindestens einem Beschleunigungsmesser stammen, und
o Extrahieren mindestens eines Niederfrequenzbereichs der Daten, die aus dem zweiten Satz von mindestens einem Beschleunigungsmesser stammen.

3. System nach einem der vorhergehenden Ansprüche, ferner beinhaltend
- Mittel zum Bestimmen der Position der Person während ihres Schlafs durch die Daten, die aus mindestens dem einen von dem ersten Satz von mindestens einem Beschleunigungsmesser und dem zweiten Satz von mindestens einem Beschleunigungsmesser stammen.

4. System nach einem der vorhergehenden Ansprüche, ferner beinhaltend mindestens einen der folgenden Sensoren:
- einen Photoplethysmographie(PPG)-Sensor,
- ein Mikrofon und
- einen Satz von mindestens einem Sensor zur Messung der Winkelposition, der mit dem ersten Satz von mindestens einem Beschleunigungsmesser synchronisiert ist und dazu konfiguriert ist, in einer Thoraxposition der Person platziert zu werden;
- wobei die Vorrichtung zum Filtern der Daten ferner zu Folgendem konfiguriert ist:
- Verknüpfen der Daten eines Satzes von mindestens einem Sensor zur Messung der Winkelposition mit den Daten von mindestens dem einen von dem ersten Satz von mindestens einem Beschleunigungsmesser und dem zweiten Satz von mindestens einem Beschleunigungsmesser, zum Extrahieren von Positionseigenschaften des Benutzers oder atypischen Winkelgeschwindigkeitsschwankungen beim Atmen; und/oder
- Mitteln der aus dem optischen Photoplethysmographie-Sensor stammenden Daten.

5. System nach einem der vorhergehenden Ansprüche, wobei die Recheneinheit dazu konfiguriert ist, einen Satz von mindestens einem Herz-Lungen-Deskriptor der Person von Folgenden zu bestimmen:
• einem Apnoe-Hypopnoe-Index (AHI),
• einem ersten Satz von Atemanstrengungen in den Daten, die aus dem ersten Satz von mindestens einem Beschleunigungsmesser stammen,
• einem zweiten Satz von Atemanstrengungen in den Daten, die aus dem zweiten Satz von mindestens einem Beschleunigungsmesser stammen,
• einem Thorax-Abdomen-Desynchronisationsindex durch einen synchronisierten Vergleich der Atemanstrengungen erster Satz Atemanstrengungen und der Atemanstrengungen zweiter Satz Atemanstrengungen,
• der Herzfrequenzvariabilität (HFV),
• dem Oximetrieprofil oder der Sauerstoffsättigung,
• der Atmungsentropie pro Zeiteinheit,
• dem Schnarchprozentsatz pro Zeiteinheit; und
• einem nächtlichen Profil der Sauerstoffsättigung.

6. System nach einem der vorhergehenden Ansprüche, wobei die Recheneinheit dazu konfiguriert ist, vor dem Vergleich einen Satz von Eigenschaften aus dem Satz der bestimmten Eigenschaften beispielsweise durch einen Algorithmus vom Typ Sequential Feature Selection (SFS) oder Fast Correlation-Based Filter (FCBF) auszuwählen.

7. System nach einem der vorhergehenden Ansprüche, wobei die Recheneinheit dazu konfiguriert ist, mindestens eines von Folgenden zu berechnen:
- der zeitlichen Korrelation zwischen mindestens zwei Ereignissen, die über einen gleichen Zeitbereich oder über mehrere Zeitbereiche bestimmt werden;
- einem Satz von Paaren (Ereignis / Position),
- einem Satz von Paaren (Ereignis / Eigenschaft),
- einem Satz von Tripeln (Ereignis / Eigenschaft / Position),
- einem Satz von Quadrupeln (Ereignis / Ereignis / Eigenschaft / Position).

8. System nach einem der vorhergehenden Ansprüche, wobei die Recheneinheit dazu konfiguriert ist, eine Ereigniskette zu bestimmen, die während eines vorbestimmten Zeitfensters zu einem Herz-Lungen-Deskriptor mit anormalem Wert geführt hat.

9. System nach einem der vorhergehenden Ansprüche, ferner beinhaltend Mittel zur Aktivierung / Deaktivierung der Beschleunigungsmesser und optionalen Sensoren.

10. System nach einem der vorhergehenden Ansprüche, wobei der Speicher zur Aufzeichnung der Daten, die aus den Beschleunigungsmessern und den optionalen Sensoren stammen, und der Speicher zur Aufzeichnung des Referenzmodells identisch sind;
wobei der Speicher:
- sich entweder entfernt befindet
- oder mit dem ersten Satz von mindestens einem Beschleunigungsmesser, dem zweiten Satz von mindestens einem Beschleunigungsmesser sowie den optionalen Sensoren eine drahtgebundene Verbindung aufweist.

11. System nach einem der vorhergehenden Ansprüche, beinhaltend:
- ein Tiefpassfilter, das dazu konfiguriert ist, eine Lungenkomponente zu extrahieren;
- ein Bandpassfilter, das dazu konfiguriert ist, eine Herzkomponente zu extrahieren; und
- ein Hochpassfilter, das dazu konfiguriert ist, eine Schnarchkomponente zu extrahieren.

12. System nach Anspruch 11, wobei:
- das Tiefpassfilter dazu konfiguriert ist, eine Hauptkomponentenanalyse (PCA) oder eine Analyse durch Diskrete Fourier-Transformation umzusetzen;
- das Bandpassfilter dazu konfiguriert ist, eine adaptive Filterung durch Hüllkurvenextraktion, durch Lernen, durch Kreuzkorrelation, durch k-Means-Clustering oder durch Hauptkomponentenanalyse (PCA) umzusetzen, und
- das Hochpassfilter dazu konfiguriert ist, eine Spektralanalyse umzusetzen.

## Claims

1. System for determining a set of at least one cardio-respiratory descriptor of an individual during sleep, comprising:
- a measuring device comprising:
o a first set of at least one accelerometer, configured so as to be placed in a thoracic position on the individual; and
o a second set of at least one accelerometer, synchronized with the first set of at least one accelerometer and configured so as to be placed in an abdominal position on said individual; and
o optionally, at least one from amongst a photoplethysmographic sensor (PPG), a microphone and a set of at least one gyroscope synchronized with the first set of at least one accelerometer;
- a memory for recording the data coming from the accelerometers and from the optional sensors;
**characterized in that** it further comprises:
- a reference model recorded in a memory, the model comprising a correspondence between a set of characteristics distributed over time and a set of given physiological events, each event preferably being representative of a potential sleep disorder;
- a device for filtering the data coming from the accelerometers configured for extracting low-frequency, medium-frequency and high-frequency ranges, and
- a computer configured for:
o determining, per extracted frequency band, a set of at least one characteristic representative of a cardio-respiratory and physiological state, together with the time at which said characteristic was extracted;
o comparing the temporal distribution of said set of at least one determined characteristic with the temporal distribution of similar characteristics coming from said reference model;
o deducing therefrom a set of at least one probable corresponding event which said individual experienced during a predetermined period of time;
o determining, based on at least one from amongst said events, a set of at least one cardio-respiratory descriptor of the individual.

2. System according to Claim 1, in which the device for filtering the data is configured for:
o extracting a low-frequency range, a medium-frequency range and a high-frequency range from the data coming from the first set of at least one accelerometer, and
o extracting at least one low-frequency range from the data coming from the second set of at least one accelerometer.

3. System according to either one of the preceding claims, furthermore comprising
- means for determining the position of said individual during sleep, from the data coming from at least one from amongst said first set of at least one accelerometer and said second set of at least one accelerometer.

4. System according to any one of the preceding claims, furthermore comprising at least one of the sensors from amongst
- a photoplethysmographic sensor (PPG),
- a microphone and
- a set of at least one sensor for measuring angular position, synchronized with the first set of at least one accelerometer and configured so as to be placed in a thoracic position on said individual;
- the device for filtering the data being furthermore configured for:
- coupling the data from a set of at least one sensor for measuring angular position with the data of at least one from amongst the first set of at least one accelerometer and the second set of at least one accelerometer, for the extraction of characteristics on position of the user or on atypical variations in respiratory angular speed; and/or
- averaging the data coming from the optical photoplethysmographic sensor.

5. System according to any one of the preceding claims, in which the computer is configured for determining a set of at least one cardio-respiratory descriptor of the individual from amongst:
• an apnea-hypopnea index (AHI),
• a first set of respiratory forces in the data coming from the first set of at least one accelerometer,
• a second set of respiratory forces in the data coming from the second set of at least one accelerometer,
• a thorax-abdomen desynchronization index, by synchronized comparison of the first set of respiratory forces and of the second set of respiratory forces,
• the heart rate variability (HRV),
• the oxymetric profile or oxygen saturation,
• the respiratory entropy per unit time,
• the percentage of snoring per unit time; and
• a night-time profile of the oxygen saturation.

6. System according to any one of the preceding claims, in which the computer is configured for selecting a set of characteristics from amongst the set of the determined characteristics, for example by an algorithm of the Sequential Feature Selection (SFS) or Fast Correlation-Based Filter (FCBF) type, prior to the comparison.

7. System according to any one of the preceding claims, in which the computer is configured for calculating at least one from amongst:
- the temporal correlation between at least two events identified over the same range of time or over several ranges of time;
- a set of (event / position) pairs,
- a set of (event / characteristic) pairs,
- a set of (event / characteristic / position) triplets,
- a set of (event / / event / characteristic / position) quadruplets.

8. System according to any one of the preceding claims, in which the computer is configured for identifying a chain of events which, during a predetermined time window, resulted in a cardio-respiratory descriptor of abnormal value.

9. System according to any one of the preceding claims, furthermore comprising means of enabling / disabling the accelerometers and optional sensors.

10. System according to any one of the preceding claims, in which the memory for recording the data coming from the accelerometers and from the optional sensors and the memory for recording the reference model are the same;
said memory being:
- either remote,
- or connected via a wired data link with the first set of at least one accelerometer, the second set of at least one accelerometer and also with the optional sensors.

11. System according to any one of the preceding claims, comprising:
- a low-pass filter, configured to extract a respiratory component;
- a bandpass filter, configured to extract a cardiac component; and
- a high-pass filter, configured to extract a snoring component.

12. System according to Claim 11, in which:
- the low-pass filter is configured to implement a principal component analysis (PCA) or an analysis based on a discrete Fourier transform;
- the bandpass filter is configured to implement adaptive filtering, via envelope extraction, learning, cross-correlation, k-mean clustering, or principal component analysis (PCA), and
- the high-pass filter is configured to implement spectral analysis.
